Europäisches Patentamt

European Patent Office

Office européen des brevets

(19)

(11) Publication number: **0 144 176 B1**

(12) **EUROPEAN PATENT SPECIFICATION**

(45) Date of publication of patent specification: **11.12.91**

(51) Int. Cl.⁵: **G01N 33/542**, G01N 33/74, G01N 33/68, G01N 33/569, G01N 33/574, G01N 33/563, G01N 33/577

(21) Application number: 84307834.6

(22) Date of filing: 13.11.84

(54) **Method of measuring a biological ligand.**

(30) Priority: **18.11.83 JP 217145/83**
**09.12.83 JP 231241/83**

(43) Date of publication of application:
**12.06.85 Bulletin 85/24**

(45) Publication of the grant of the patent:
**11.12.91 Bulletin 91/50**

(84) Designated Contracting States:
**CH DE FR GB IT LI NL**

(56) References cited:
**EP-A- 0 084 807**
**EP-A- 0 095 089**
**EP-A- 0 119 767**
**WO-A- 8/21378**
**US-A- 3 935 074**

(73) Proprietor: **FUJIREBIO KABUSHIKI KAISHA also trading as FUJIREBIO INC.**
**6-7, Shimoochiai 4-chome Shinjuku-ku Tokyo 161(JP)**

(72) Inventor: **Ashihara, Yoshihiro**
**2-402, Fuchudanchi 28-1, Harumicho, 1-chome**
**Fuchu-shi Tokyo(JP)**
Inventor: **Kasahara, Yasushi**
**310, 4-4, Nagayama 3-chome**
**Tama-shi Tokyo(JP)**

(74) Representative: **Silverman, Warren et al**
**HASELTINE LAKE & CO. Hazlitt House 28 Southampton Buildings Chancery Lane London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 144 176 B1

## Description

This invention relates to a method of measuring a biological ligand.

The ability to measure the concentration in the blood of a drug administered to a patient is an important factor in the treatment of the patient. Moreover, the detection in the blood of trace components derived from various diseases is important in early diagnosis of the diseases in patients. As these medicinal substances and trace components derived from various diseases can be bound to biological ligands, it is important to be able to measure such biological ligands which incorporate medicinal substances and trace substances derived from various diseases.

A humoral fluid such as blood contains various components, and some of them are similar to each other in their molecular weights and/or physiological properties. For this reason, the measurement of such components requires high specificity and high sensitivity. Furthermore, in order to utilise conventional inspection techniques for diagnosis of a disease, the measuring procedure is of necessity to be simple.

Various methods of detecting these trace components in blood have been developed, among which enzyme immunoassay is widely employed because of its superior sensitivity and specificity and its ability to allow rapid treatment of a large number of samples. However, the sensitivity of conventional enzyme immunoassay techniques is not sufficient and it is not easy to obtain an exact measurement of concentration because of the complicated washing procedures that are used and the transferring of tubes.

The present inventors have investigated various enzyme immunoassay methods using various combinations of a ligand or an antibody and an enzyme or a material having an enzyme inhibitory activity as follows:

There is described in Japanese published patent application No.142466/1984 a method of measuring a biological ligand which comprises, contacting a ligand (1) to be measured and a covalently coupled combination of a ligand (2) having an antigenic determinant common to one of the antigenic determinant(s) of the above ligand (1) and biotin or a derivative thereof capable of reacting with avidin or streptoavidin as biotinyl enzyme inhibitor, with an antibody capable of reacting with the above common antigenic determinant in an aqueous solution, contacting the above covalently coupled combination with a biotinyl enzyme inhibitor such as avidin, streptoavidin or one of their derivatives capable of reacting with biotin, and measuring the residual biotinyl enzyme activity.

In the method of EP-A-0119767 for measuring a biological ligand, there are brought into contact with each other in aqueous solution (a) a ligand to be measured, (b) an enzyme or a covalently coupled combination of an enzyme and a macromolecular substance, and (c) either a covalently coupled combination of an antibody against the above ligand and an antibody against the above enzyme, or a covalently coupled combination of an antibody against the above ligand, an antibody against the above enzyme and a macromolecular substance, determining the residual activity of the enzyme and utilising the result obtained as a measure of the ligand.

In our EP-A-0124366, there is disclosed a method of measuring a biological ligand, characterised by bringing into contact with each other in aqueous solution:

(A) a biologically active composition comprising firstly an immobilized antibody phase whose antibody is capable of reacting with a ligand (1) to be measured or an immobilised phase of a biological ligand (2) which is capable of reacting with said antibody and secondly an immobilised biotinyl enzyme phase or immobilized biotinyl enzyme inhibitor phase,

(B) a water-soluble material covalently coupled combination of whichever of said ligand (2) or said antibody on the one hand and a biotinyl enzyme inhibitor or a biotinyl enzyme on the other hand are absent from the biologically active composition, the biotinyl enzyme inhibitor then being one which is capable of reacting with the biotinyl enzyme of the biologically active composition or the biotinyl enzyme then being one which is capable of reacting with the biotinyl enzyme inhibitor of the biologically active composition, and

(C) the ligand (1) to be measured, and measuring the residual biotinyl enzyme activity or the residual biotinyl enzyme inhibitory activity of said biologically active composition or said aqueous solution and utilizing the result as a measure of the ligand.

Finally in our EP-A- 0132292 (European Patent Application No.84304093.2), there is described a method of measuring a biological ligand which comprises bringing into contact with each other in aqueous solution (A) a ligand (1) to be measured and, (B) a covalently coupled combination of a biotinyl enzyme inhibitor and a ligand (2) having an antigenic determinant which is also an antigenic determinant of ligand (1) and (C) an antibody capable of reacting with the said antigenic determinant, adding a biotinyl enzyme, measuring the residual biotinyl enzyme activity and utilising the measurement thereby obtained as a measure of the biological ligand.

It is an object of this invention to provide a yet further method of measuring a biological ligand, which possesses high sensitivity and simplicity of operation.

This invention is based on the discovery that when a covalently-coupled combination of an enzyme capable of acting on a water-insoluble macromolecular substance and a ligand or an antibody is employed as the covalently coupled combination of substances and brought into contact with the ligand to be measured, the ligand in a test sample can be detected to a high degree of sensitivity and the operation is relatively simple to carry out.

According to the present invention, there is thus provided a method of measuring a biological ligand which comprises, bringing into contact with each other in aqueous solution (A) a ligand (1) to be measured and (B) either a covalently coupled combination (1) of an antibody against the ligand (1) and an enzyme capable of acting on a water-insoluble macromolecular substrate or a covalently coupled combination (2) of a said enzyme and a ligand (2) having an antigenic determinant which is an antigenic determinant of said ligand (1), which covalently coupled combination (2) is present in the aqueous medium together with a said antibody, said antibody reacting (c) with said ligand (1) or (b) with said ligand (1) and with said ligand (2) to which said enzyme is covalently coupled digesting with said enzyme (C) a said water insoluble macromolecular substrate, determining the residual activity of the enzyme after it has acted on said macromolecular substrate and utilising the measurement thereby obtained as a measure of the biological ligand.

The subject to be measured by the method of the invention is termed herein ligand (1). The ligand (1) will be a substance having one or more antigenic determinants, and includes, for example, hormones derived from various endocrine glands, plasma proteins such as immunoglobulin, albumin and ferritin, viral antigens such as HB antigen, bacteria, α-fetoprotein, and carcinoembryonic antigens. This ligand (1) may also be a hapten or may be a first antibody in the double antibody method. The ligand (1) may furthermore be the conjugate of the antigen to be measured and a first antibody as in the double antibody method. The fluid medium acting as the source of ligand (1) may be inter alia, serum or urine.

The ligand (2) which is optionally used will also have one or more antigenic determinants and at least one antigenic determinant must be an antigenic determinant of the ligand (1). All the antigenic determinants of ligand (2) may be antigenic determinants of ligand (1), and accordingly, ligand (2) may be identical with the ligand (1).

The antibody which is used should be the antibody against this common antigenic determinant when two ligands are present in the aqueous medium being used. The antibody whether or not a ligand (2) is used may include fragments of immunoglobulin such as F(ab')₂, Fab' and Fab.

Such an antibody may be produced by following any suitable method for producing an antibody. For example, the ligand (1), the ligand (2) or a covalently coupled combination of either of these ligands and a protein material is injected once or several times into the subcutaneous region of the back, foot pad or femoral muscle of a warm-blooded animal such as a rabbit, goat, horse, guinea pig or chicken, in an amount of from 0.3 to 2 mg per kg together with an adjuvant to produce the antibody in a humoral fluid such as serum. This humoral fluid may be used as it is as the antibody, although, the antibody is preferably separated by following a conventional immunoglobulin isolation method.

Alternatively, the antibody may be produced as a monoclonal antibody. In this case, one of the above antigens is injected several times into the abdominal cavity of a mouse together with an adjuvant, and the spleen of the mouse is excised. The spleen cell is fused with a mouse myeloma cell by a conventional method involving the use of polyethylene glycol. The hybridoma thus obtained is cultured and cloned, and the cell which is capable of producing the desired antibody is obtained. This cell is injected into the abdominal cavity of a mouse, and multiplied. Then, ascites are collected, and the desired antibody is separated from the ascites.

Should the antibody not be covalently coupled combined with the enzyme, as will be described hereinafter and should the enzyme activity not vary appreciably through the reaction of the antibody with the ligand (2) portion of the covalently coupled combination which is also described hereinafter, a macromolecular compound may preferably be bound to the antibody prior to use. Preferred macromolecular compounds for this purpose are water-soluble, and their molecular weights are greater than 100,000 daltons. Examples of such macromolecular compounds include polysaccharides and their derivatives such as soluble dextran, carboxymethyl dextran, dextran with induced amino group and amylose, proteins such as gelatin, hemocyanin and ferritin, and polyethylene glycol The macromolecular compound may be covalently coupled to the antibody by one of the coupling methods described hereinafter.

The enzyme is able to act on a water-insoluble macromolecular substrate (C). Enzymes whose activities are easily measured are preferably used. Examples of such enzymes include α-amylase, cellulase, collagenase, mannase, protease, elastase and lipase.

The enzyme is initially covalently coupled with the antibody or the ligand (2). When it is covalently coupled with the antibody, ligand (2) is not used. On the other hand, when it is covalently coupled with ligand (2), the antibody may be used in its natural form or covalently coupled with a macromolecular compound.

The method of covalently coupling the enzyme and the antibody or the ligand (2) may be selected in accordance with the functional groups of substances to be combined. Such functional groups include, amino groups, carboxyl groups, hydroxyl groups, thiol groups, imidazole groups and phenyl groups. The coupling of amino groups may be carried out by many methods such as the diisocyanate method, the glutaraldehyde method, the difluorobenzene method, and the benzoquinone method. When coupling an amino group and a carboxyl group, it is possible to use the peptide-binding method using a carboxyl group coupled to a succinimido ester and the carbodiimide method or to use Woodward reagent. The periodate oxidation method (Nakane method) where a bridge is formed between amino groups and sugar chains may also be utilised. When using a thiol group for the aforesaid binding, a carboxyl group may be first converted to a succinimido ester group, and this ester group is then allowed to react with cysteine to introduce the thiol group thereof. The two thiol groups are then linked by using a thiol-reactive bifunctional cross-linking reagent such as phenylene- bismaleimide. Typical of methods utilising a phenyl group are the diazotization method and the alkylation method. In addition to the foregoing methods, it is possible to use a suitable method selected from the various methods described in "Methods in Immunology and Immunochemistry" (C.A. Williams et al., 1976, Academic Press N.Y.). and "Koso Meneki Sokutei-ho" (E. Ishikawa et al., 1978, Igaku-shoin (Japan)). The molar ratio of components used in forming the covalently coupled combination is not limited to 1:1, and other suitable ratios can be easily selected. After the coupling reaction, the covalently coupled combination produced is purified by gel filtration, ion-exchange chromatography and affinity chromatography,and lyophilized, if desired.

When the enzyme is covalently coupled with the antibody, the antibody thus combined is then brought into contact with the ligand (1) to be measured. When the enzyme is covalently coupled with the ligand (2), the antibody is first brought into contact with the ligand (1) and the ligand (2) in its covalently coupled combination with the enzyme. In the latter case, the order of contacting is not limited, and either the ligand (1) or the covalently coupled combination of enzyme and ligand (2) may first be allowed to contact the antibody. Of course, both ligand (1) and the covalently coupled combination of enzyme and ligand (2) may be allowed to contact the antibody at the same time. The temperature of the aqueous solution is usually kept at from 20 to 45°C, and the pH is usually kept at from 4 to 8.5. In order to keep the pH constant, a buffer solution such as a phosphate buffer solution or an acetate buffer solution may be employed. Since the amounts of the covalently coupled combination of enzyme and ligand (2) or the covalently coupled combination of enzyme and antibody, as the case may be, the antibody, when used on its own, will vary, inter alia, according to their character, the character of ligand (1) and the contacting conditions, the amounts are preferably determined by a preliminary test.

If the antibody is not covalently coupled with the enzyme and if the enzyme activity has been found not to vary appreciably through the reaction of the antibody with the ligand (2) portion of the covalently coupled combination of enzyme and ligand (2), a second antibody may be reacted with the antibody covalently coupled to the ligand (2) portion. The second antibody may be prepared by following the method previously described herein.

Subsequently, the ligand (1), the covalently coupled combination of substances and, when appropriate, antibody are brought into contact in an aqueous medium with a water-insoluble macromolecular (C). The covalently coupled combination may initially be in the reaction solution to which the water-insoluble macromolecular substance is added or it may also be separate from the reaction solution, too.

The macromolecular (C) is then subjected to the action of the enzyme, and it is usually a conventional substrate for the enzyme. The macromolecular substance is characterised by its being insoluble in water. Hence the interacting of the macromolecular substance and the enzyme portion of the covalently coupled combination thereof with ligand(s) and antibody is carried out at the boundary between solid and solution, and as a result, steric hindrance of macromolecule(s) bound to the enzyme is a major factor. This steric hindrance is supported by an experiment which has been carried out wherein a pentose and an insoluble starch were digested by an $\alpha$-amylase which was in its natural form or which had previously been allowed to combine with a macromolecule. The results of the experiment showed that the enzyme activity was scarcely lowered by the covalent coupling of the enzyme with the macromolecule when the substrate was pentose, while it was considerably lowered when the substrate was the insoluble starch. Macromolecular substance/enzyme pairings which can be used include insoluble starches in the case of $\alpha$-amylase, celluloses in the case of cellulase, collagen in the case of collagenase, mannan in the case of mannase, insoluble proteins in the case of proteases, elastin in the case of elastase, and various lipids in the case of lipases. When a macromolecular substance is normally water-soluble, it may also be used in the method of this invention if it has been subjected to insolubilisation. The insolubilisation may be carried out by covalent coupling with an insoluble carrier material or by subjecting the macromolecular substance to homopolymerization.

The conditions under which the enzyme reaction will be carried out will depend on the enzyme being employed.

After the reaction, the residual magnitude of the activity of the enzyme prior to its acting on the macromolecular substance will be determined by detecting changes in the reaction mixture, such as the concentration of a decomposition product or the decrease in the substrate for the enzyme.

The method of this invention enables a biological ligand to be detected and determined in high sensitivity and in high specificity. The operation of this method is simple, and a biological ligand can easily and inexpensively be determined. There are essentially no restrictions on the ligand to be measured, although the present method is especially suitable for use when measuring ligands having relatively high molecular weights. When using a covalently coupled combination of the antibody and enzyme, since the ligand is used only as the antigen which is necessary to produce the antibody, the amount of the ligand present may be very small. Accordingly, this method is effective when only a small amount of the ligand can be obtained and when the ligand is extremely expensive.

The following Examples illustrate this invention:

## EXAMPLE 1

i) Preparation of Cellulose Substrate

A piece of filter paper 20 cm x 20 cm was cut. The piece was immersed in a reactive blue solution prepared by dissolving 5 g of reactive blue and 5 g of $Na_2CO_3$ in 200 ml of distilled water, and kept at 60°C for 3 days with occasional stirring. The filter paper was then sufficiently washed with distilled water to remove the excess dye. Subsequently, the filter paper was dried in a dryer having a thermostat, and cut into pieces sized 1 cm x 5 cm which were to be the substrate for cellulase.

ii) Preparation of Covalently Coupled Combination of Cellulose and Human Human IgG

10 mg of cellulase were dissolved in 2 ml of 0.1 M phosphate buffer solution of pH 6.0, and 200 $\mu$l of a 2 mg/ml 4-maleimidomethyl cyclohexane-1-carboxylic acid succinimido ester (CHMS) dimethyl sulphoxide solution were added. The mixture was allowed to stand at ambient temperature for one hour. Gel filtration using Sephadex G-25 (Sephadex is a Registered Trade Mark) was carried out and unreacted CHMS was removed from the reaction mixture. The reaction mixture was then concentrated to 1 ml to obtain CHM-induced cellulase.

Separately, 10 mg of human IgG were dissolved in 2 ml of 0.1 M phosphate buffer solution of pH 7.5. 200 $\mu$l of a 9 mg/ml S-acetylmercaptosuccinic anhydride (SAMS) dioxane solution were added to this, and allowed to stand at 37°C for 1 hour. Subsequently, 200 $\mu$l of 1 M hydroxylamine aqueous solution of pH 7.5 were added, and the reaction mixture obtained was allowed to stand at 37°C for 30 minutes. Gel filtration using Sephadex G-25 was carried out, and unreacted SAMS was removed from the reaction mixture. HS-human IgG solution thus obtained was added to 1 ml of the above CHM-induced cellulase, and allowed to stand at 37°C for 2 hours. The reaction mixture was subjected to gel filtration using Sephacryl S-300 (Sephacryl is a Registered Trade Mark) and in this way the target combination of cellulase and human IgG was obtained.

iii) Measurement of Human IgG

50 $\mu$l of a standard solution containing human IgG were added to 50 $\mu$l of a solution containing the above covalently coupled combination of enzyme (cellulase) and ligand (human IgG). 5 l of anti-human IgG goat serum were added to this mixture, and allowed to stand at 37°C for 1 hour. 1 ml of 0.1 M acetate buffer solution pH 5.0 was added to this, and one piece of the blue cellulose filter paper prepared in step i) was then added. After 1 hour, the absorbance at 620 nm of the reaction solution was measured. The relationship between the human IgG concentrations in standard solutions of different concentration and the absorbance is shown in the following Table 1.

Table 1

| Human IgG (μg) | A$_{620}$ nm |
|---|---|
| 0 | 0.280 |
| 100 | 0.380 |
| 200 | 0.490 |
| 400 | 0.630 |
| 800 | 0.990 |
| 2000 | 1.220 |

These results were suitable for plotting as a standard curve to enable the human IgG concentration of solutions of unknown concentration to be determined.

EXAMPLE 2

i) Preparation of CHM-induced Amylase

5 mg of Bacillus subtilis amylase were dissolved in 1 ml of 0.1 M phosphate buffer solution of pH 6.3. 100 μl of 2 mg/ml CHMS dimethylformamide (DMF) solution were added and the reaction mixture was allowed to stand at ambient temperature for 1 hour. The reaction mixture was then introduced into a Sephadex G-25 column, and gel filtration was carried out by using 0.1 M phosphate buffer solution of pH 6.3. The void fractions were collected to obtain the target CHM-induced amylase.

ii) Preparation of SH-induced α-Fetoprotein

5 mg of α-fetoprotein were dissolved in 0.1 M phosphate buffer solution containing 5 mM EDTA. 100 μl of 9 mg/ml SAMS DMF solution was added to this, and allowed to react at 37°C for 1 hour. 110 μl of 1M aqueous hydroxylamine solution of pH 7.5 were added to the reaction mixture which was allowed to warm up at 37°C for 30 minutes. Subsequently, gel filtration of the reaction mixture using Sephadex G-25 was carried out and the void fractions were collected to obtain the target SH-induced α-fetoprotein.

iii) Preparation of Covalently Coupled Combination of Amylase and α-Fetoprotein

The CHM-induced amylase solution produced in step i) was mixed with the SH-induced α-fetoprotein solution from step ii). The mixture was concentrated to 1 ml, and allowed to react at 4°C overnight. The reaction solution was introduced into a Sephacryl SD-300 column, and gel filtration was carried out by using 20 mM phosphate buffered saline solution of pH 7.0. The fractions containing the covalently coupled combination of enzyme and ligand in a molar ratio of 1:1 were collected.

iv) Measurement of α-Fetoprotein

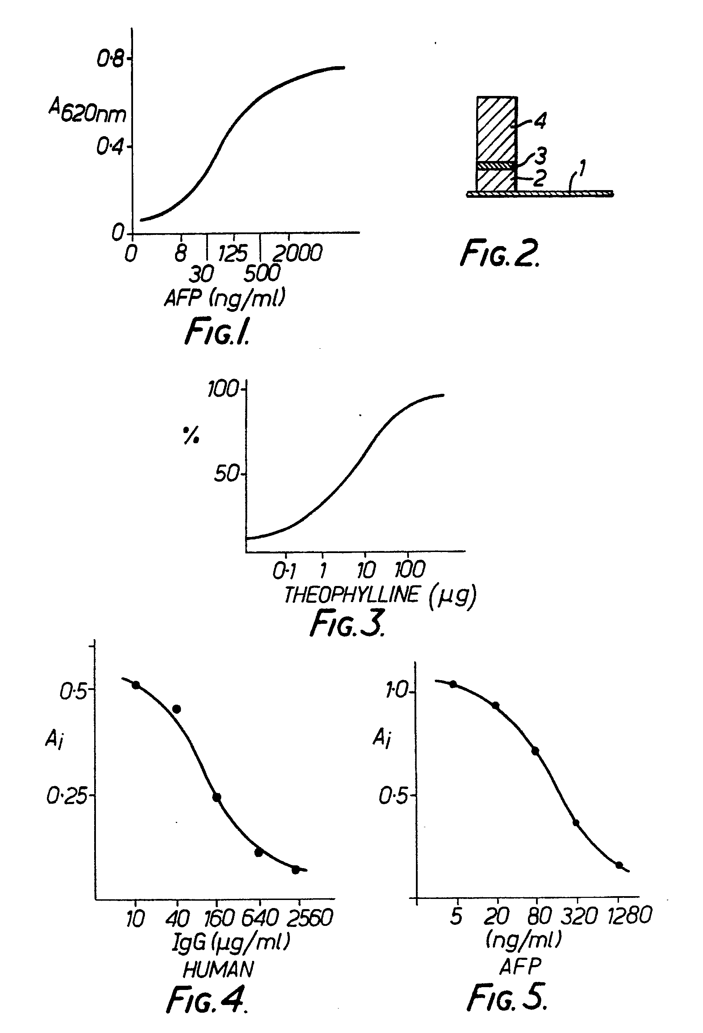

50 μl samples of α-fetoprotein solutions whose concentrations were in the range of 0-2000 ng were each mixed with 50 μl of the covalently coupled combination solution prepared in step iii). 50 μl of 8 μg/ml anti-α-fetoprotein goat IgG solution were added to each mixture, and allowed to react for 20 minutes. 1.0 ml of a suspension of blue starch (made by Pharmacia, Diagnostics, A.B.) was added to each reaction mixture, and allowed to react at 37°C for 20 minutes. The enzyme reaction was terminated by adding 1 ml of 0.5 N NaOH. The mixture was stirred, and then centrifuged at 3,500 rpm for 2 minutes. The absorbance at 620 nm of the supernatant was measured. The relationship between the α-fetoprotein concentration and the absorbance thus obtained is shown in Figure 1 of the accompanying drawings which provides a standard curve for determining unknown α-fetoprotein concentrations in body fluids.

EXAMPLE 3

5 mg of α-amylase were dissolved in 1 ml of 0.1 M carbonate buffer solution of pH 8.0. 100μl of 20 μg/ml 3-carboxytheophylline succinimido ester DMF solution were added to this, and allowed to react at ambient temperature for 1 hour. Gel filtration using a Sephadex G-25 column which had previously been equilibrated with 20 mM phosphate buffered saline solution of pH 6.5 containing 20 mM calcium chloride was carried out, and the void fractions were collected. The fractions were concentrated to 1 ml to obtain an enzyme/ligand covalently coupled combination of theophylline and α-amylase.

50 μl of 800 mg/ml of this covalently coupled combination solution were added to 50 μl of serum, and in order to inhibit human serum amylase, 50 μl of 500 μg/ml anti-human amylase goat IgG were added to the mixture. 50 μl of 15 μg/ml anti-theophylline mouse IgG was also then added, and allowed to react at 37°C for 30 minutes. 100 μl of this reaction solution were dropped onto a laminate film which consisted of a polystyrene film 1, a cation-exchange resin layer 2, a reflection layer 3 and a blue starch layer 4 as shown in Figure 2 of the accompanying drawings. The amylase activity at ambient temperature after 20 minutes was measured by using a reflectometer. This procedure was repeated, but with the inclusion of theophylline in different concentrations in the reaction solution so that residual amylase activity decreased as theophylline concentration increased. The relationship between the theophylline concentration and the reflection intensity thus obtained as a measure of enzyme activity is shown in Figure 3 of the accompanying drawings which is thus a standard curve for use to determine the theophylline concentration in a test specimen of serum of unknown theophylline concentration.

## EXAMPLE 4

i) Preparation of Cellulase Substrate
   Blue cellulose filter paper samples sized 1 cm x 5 cm were prepared in the same manner as described in step i) of Example 1.
ii) Preparation of Covalently Coupled combination cellulase and Anti-Human IgG Goat IgG
   CHMS-induced cellulase 1 ml was prepared in the same manner as described in the first part of step ii) of Example 1.
   Separately, 10 mg of anti-human IgG goat IgG were dissolved in 2 ml of 0.1 M phosphate buffer solution containing 5 mM EDTA. 200 $\mu$l of 9 mg/ml SAMS dioxane solution were added to this, and allowed to stand at 37°C for 1 hour. 200 $\mu$l of 1 M hydroxylamine aqueous solution of pH 7.5 were added, and the reaction mixture was allowed to stand at 37°C for 30 minutes. The reaction mixture was then subjected to gel filtration using Sephadex G-25, and unreacted SAMS was removed.
   This HS-anti-human IgG goat IgG solution was added to 1 ml of the aforesaid CHM-induced cellulase, and allowed to stand at 37°C for 2 hours. The reaction mixture was separated by gel filtration using Sephacryl SD-300, and the target enzyme/antibody covalently coupled combination of cellulase and anti-human IgG goat IgG was obtained.
iii) Measurement of Human IgG
   50 $\mu$l of a standard solution containing human IgG were added to 50 $\mu$l of a solution containing the above covalently coupled combination of cellulase and anti-human IgG goat IgG, and allowed to stand at 37°C for 30 minutes. 1 ml of 0.1 M acetate buffer solution pH 5.0 was added, and one piece of the blue cellulose filter paper prepared in step i) was then added. After 1 hour, the absorbance at 620 nm of the reaction solution was measured. The relationship between the human IgG concentration and the absorbance for standard solutions of different human IgG concentration is shown in Figure 4 of the accompanying drawings which is thus a standard curve for enabling unknown human IgG concentrations in fluids to be determined.

## EXAMPLE 5

i) Preparation of CHM-induced Amylase
   CHM-induced amylase was prepared from <u>Bacillus</u> <u>subtilis</u> amylase in the manner as described in step i) of Example 2.
ii) Preparation of Anti-Human $\alpha$-Fetoprotein Goat IgG F(ab')$_2$
   10 mg of anti-human $\alpha$-fetoprotein goat IgG were dissolved in 2 ml of 0.1 M acetate buffer solution of pH 4.0. 300 $\mu$g of pepsin were added to this, and stirring at 37°C for 18 hours was effected. The solution was adjusted to pH 6.0 by adding 0.1 N NaOH, and introduced into a Sephacryl S-300 column which had been previously equilibrated with 0.1 M phosphate buffered 1 mM EDTA solution of pH 6.3, and eluted using the above phosphate buffer solution without EDTA. Peak fractions corresponding to a molecular weight of about 100,000 were collected, and concentrated to 1 ml to obtain the target anti-human $\alpha$-fetoprotein goat IgG F(ab')$_2$.
iii) Preparation of Covalently Coupled Combination of Amylase and Anti-Human $\alpha$-Fetoprotein Goat IgG Fab'
   1 ml of 0.1 M phosphate buffered 1 mM EDTA solution of pH 6.0 containing 6 mg of the above anti-human $\alpha$-fetoprotein goat IgG F(ab')$_2$ were mixed with 100 $\mu$l of 10mg/ml aqueous 2-mercaptoethylamine hydrochloride solution, and stirred at 37°C for 90 minutes. Gel filtration using a Sephadex G-25 column which had been previously equilibrated with 0.1 M phosphate buffer solution of pH 6.3 was carried out, and unreacted 2-mercaptoethylamine was removed to obtain HS-Fab'. 2 mg of CHM-induced $\alpha$-amylase prepared in step i) were added, and reaction was allowed to take place at 37°C for 90 minutes. Subsequently, this reaction mixture was separated by gel filtration using a Sephacryl S-300 column which was equilibrated with 0.1 M acetate buffered 5 mM calcium chloride solution of pH 6.0, and the fractions corresponding to the molecular weights of greater than 200,000 were collected. The fractions were concentrated to obtain the target covalently coupled combination of enzyme and antibody.

iv) Measurement of α-Fetoprotein

50 μl samples of α-fetoprotein solutions whose concentrations were in the range of 0-2000 ng were each mixed with 50 μl of the covalently coupled combination solution prepared in the above step iii), and allowed to react for 20 minutes. 1.0 ml of a suspension of blue starch (made by Pharmacia Diagnostics, A.B.) was added to the reaction mixture, and reaction was allowed to take place at 37°C for 20 minutes. The enzyme reaction was terminated by adding 1 ml of 0.5 N NaOH. The reaction mixture was then stirred and centrifuged at 3,500 rpm for 2 minutes. The absorbance at 620 nm of the supernatant was measured in each case and enabled the relation between the α-fetoprotein concentration and the absorbance which is shown in Figure 5 of the accompanying drawings to be obtained. This provided a standard curve for allowing the α-fetoprotein concentration of specimen fluids to be determined.

## Claims

1. A method of measuring a biological ligand which comprises, bringing into contact with each other in an aqueous solution (A) a ligand (1) to be measured and (B) either a covalently coupled combination (1) of an antibody against the ligand (1) and an enzyme capable of acting on a water-insoluble macromolecular substrate or a covalently coupled combination (2) of a said enzyme and a ligand (2) having an antigenic determinant which is an antigenic determinant of said ligand (1), which covalently coupled combination (2) is present in the aqueous medium together with a said antibody, said antibody reacting (a) with said ligand (1) or (b) with said ligand (1) and with said ligand (2) to which said enzyme is covalently coupled, digesting with said enzyme (C) a said water insoluble macromolecular substrate, determining the residual activity of the enzyme after it has acted on said macromolecular substrate and utilising the measurement thereby obtained as a measure of the biological ligand.

2. The method of claim 1, wherein a covalently coupled combination of said antibody and said enzyme is brought into contact with said ligand (1) in the aqueous medium and the enzyme of said covalently coupled combination is then brought into contact with said macromolecular substance in the aqueous medium.

3. The method of claim 1, wherein a covalently coupled combination of said enzyme and said ligand (2) is brought into contact with said antibody and said ligand (1) in the aqueous medium and the enzyme of said covalently coupled combination is then brought into contact with said water insoluble macromolecular substrate in the aqueous medium.

4. The method of claim 3, wherein said antibody is covalently coupled with a macromolecular compound.

5. The method of claim 4, wherein the macromolecular substance is water-soluble and has a molecular weight which is greater than 100,000 daltons.

6. The method of claims 1 and 3, wherein said ligand (1) and said ligand (2) is a hormone derived from an endocrine gland, a plasma protein, a viral antigen, a bacterial species, α-fetoprotein, a carcinoembryonic antigen, a hapten, a first antibody in the double antibody method or a conjugate of an antigen and a first antibody in the double antibody method.

7. The method of any one of claims 1 and 3 to 6, wherein said ligand (1) and said ligand (2) are the same substance.

8. The method of any preceding claim, wherein said antibody is a fragment of immunoglobulin.

9. The method of any one of claims 1 to 7, wherein said antibody is a monoclonal antibody.

10. The method of any preceding claim, wherein said enzyme is selected from α-amylase, cellulase, collagenase, mannase, proteases, elastase and lipases.

11. The method of claim 10, wherein said macromolecular substance is insoluble starch when the enzyme

EP 0 144 176 B1

is α-amylase, a cellulosic material when the enzyme is cellulase, collagen when the enzyme is collagenase, mannan when the enzyme is mannase, an insoluble protein when the enzyme is a protease, elastin when the enzyme is elastase, and a lipid when the enzyme is a lipase.

12. A method of measuring theophylline (1) in a sample of human serum containing amylase (2) which comprises bringing into contact with each other the serum sample containing the theophylline, anti-theophylline mouse IgG as antibody (3) against the theophylline, a covalently coupled combination of α-amylase and theophylline (4) and anti-human amylase goat IgG as an amylase inhibitor (5) whose inhibiting activity against human serum amylase (2) is stronger than that against said α-amylase, said antibody (3) reacting with the theophylline of said sample and the theophylline to which the α-amylase is covalently coupled and said amylase inhibitor (5) reacting with said amylase (2), digesting starch with the α-amylase and determining the residual activity of said α-amylase and utilizing the measurement thereby obtained as a measure of the theophylline in said human serum sample.

**Revendications**

1. Méthode de mesure d'un ligand biologique, consistant à mettre en contact les uns avec les autres, dans une solution aqueuse, (A) un ligand (1) à mesurer et (B) soit une combinaison couplée par covalence (1) d'un anticorps contre le ligand (1) et d'une enzyme capable d'agir sur un substrat macromoléculaire insoluble dans l'eau, ou une combinaison couplée par covalence (2) de ladite enzyme et d'un ligand (2) ayant un déterminant antigénique qui est un déterminant antigénique dudit ligand (1), laquelle combinaison couplée par covalence (2) est présente dans le milieu aqueux avec ledit anticorps, ledit anticorps réagissant (a) avec ledit ligand (1) ou (b) avec ledit ligand (1) et avec ledit ligand (2) auquel ladite enzyme est couplée de façon covalente, à faire digérer par ladite enzyme (C) ledit substrat macromoléculaire insoluble dans l'eau, à déterminer l'activité résiduelle de l'enzyme après qu'elle a agi sur ledit substrat macromoléculaire et utiliser la mesure ainsi obtenue comme mesure du ligand biologique.

2. Méthode de la revendication 1, dans laquelle on met une combinaison couplée par covalence dudit anticorps et de ladite enzyme en contact avec ledit ligand (1) en milieu aqueux, et on met ensuite l'enzyme de ladite combinaison couplée par covalence en contact avec ledit substrat macromoléculaire en milieu aqueux.

3. Méthode de la revendication 1, dans laquelle on met une combinaison couplée par covalence de ladite enzyme et dudit ligand (2) en contact avec ledit anticorps et ledit ligand (1) dans le milieu aqueux, et on met ensuite l'enzyme de ladite combinaison couplée par covalence en contact avec ledit substrat macromoléculaire insoluble dans l'eau, en milieu aqueux.

4. Méthode de la revendication 3, dans laquelle ledit anticorps est couplé par covalence avec un composé macromoléculaire.

5. Méthode de la revendication 4, dans laquelle le composé macromoléculaire est hydrosoluble et a une masse moléculaire supérieure à 100000 daltons.

6. Méthode des revendications 1 et 3, dans laquelle ledit ligand (1) et ledit ligand (2) sont une hormone provenant d'une glande endocrine, une protéine plasmatique, un antigène viral, une espèce bactérienne, une α-foetoprotéine, un antigène carcinoembryonnaire, un haptène, un premier anticorps dans une méthode à double anticorps ou un conjugué d'un antigène et d'un premier anticorps dans une méthode à double anticorps.

7. Méthode de l'une quelconque des revendications 1 et 3 à 6, dans laquelle ledit ligand (1) et ledit ligand (2) sont une même substance.

8. Méthode de l'une quelconque des revendications précédentes, dans laquelle ledit anticorps est un fragment d'immunoglobuline.

9. Méthode de l'une quelconque des revendications 1 a 7, dans laquelle ledit anticorps est un anticorps monoclonal.

9

10. Méthode de l'une quelconque des revendications précédentes, dans laquelle ladite enzyme est choisie parmi: α-amylase, cellulase, collagénase, mannase, proteases, élastase et lipases.

11. Méthode de la revendication 10, dans laquelle ledit composé macromoléculaire est l'amidon insoluble quand l'enzyme est l'α-amylase, un matériau cellulosique quand l'enzyme est la cellulase, le collagène quand l'enzyme est la collagénase, le mannane quand l'enzyme est la mannase, une protéine insoluble quand l'enzyme est une protéase, l'élastine quand l'enzyme est l'élastase, et un lipide quand l'enzyme est une lipase.

12. Méthode de mesure de la théophylline (1) dans un échantillon de sérum humain contenant de l'amylase (2), qui consiste à mettre en contact les uns avec les autres l'échantillon de sérum renfermant la théophylline, de l'IgG de souris anti-théophylline comme anticorps (3) contre la théophylline, une combinaison couplée par covalence d'α-amylase et de théophylline (4) et de l'IgG de chèvre anti-amylase humaine, comme inhibiteur d'amylase (5) dont l'activité d'inhibition contre l'amylase du sérum humain (2) est plus forte que celle contre ladite α-amylase, ledit anticorps (3) réagissant avec la théophylline de l'échantillon et avec la théophylline à laquelle l'α-amylase est couplée par covalence, et ledit inhibiteur d'amylase (5) réagissant sur ladite amylase (2), à faire digérer de l'amidon par l'α-amylase et à déterminer l'activité résiduelle de ladite α-amylase et utiliser la mesure ainsi obtenue comme mesure de la théophylline dans ledit échantillon de sérum humain.

## Patentansprüche

1. Verfahren zur Messung eines biologischen Liganden, umfassend das miteinander Inberührungbringen in einer wässerigen Lösung von (A) einem zu bestimmenden Liganden (1) und (B) entweder einer kovalent verbundenen Kombination (1) von einem Antikörper gegen den Liganden (1) und einem Enzym, das auf ein wasserunlösliches makromolekulares Substrat einzuwirken vermag, oder einer kovalent verbundenen Kombination (2) aus dem Enzym und einem Liganden (2) mit einer als Antigen wirkenden Determinante, die antigenisch für den Liganden (1) ist, wobei die kovalent verbundene Kombination (2) in dem wässerigen Medium zusammen mit dem Antikörper vorhanden ist, zur Reaktion bringen des Antikörpers (a) mit dem Liganden (1) oder (b) mit dem Liganden (1) und dem mit dem Enzym kovalent verbundenen Liganden (2), Digerieren von dem wasserunlöslichen makromolekularen Substrat mit dem Enzym (C), Ermittlung der restlichen Aktivität des Enzyms nach dem Einwirken auf das makromolekulare Substrat und Vewendung der so erhaltenen Messung als Maß für den biologischen Liganden.

2. Verfahren gemäß Anspruch 1, wobei eine kovalent verbundene Kombination des Antikörpers und des Enzyms in dem wässerigen Medium in Berührung gebracht wird und danach das Enzym mit der kovalent verbundenen Kombination in dem wässerigen Medium mit der makromolekularen Substanz kontaktiert wird.

3. Verfahren gemäß Anspruch 1, wobei eine kovalent verbundene Kombination vom Enzym und Liganden (2) in Berührung gebracht wird mit dem Antikörper und dem Liganden (1) in dem wässerigen Medium und das Enzym der kovalent verbundenen Kombination mit dem wasserunlöslichen makromolekularen Substrat im wässerigen Medium kontaktiert wird.

4. Verfahren gemäß Anspruch 3, wobei der Antikörper kovalent mit einer makromolekularen Verbindung verbunden ist.

5. Verfahren gemäß Anspruch 4, wobei die makromolekulare Substanz wasserlöslich ist und ein Molekulargewicht von mehr als 100 000 Dalton aufweist.

6. Verfahren gemäß den Ansprüchen 1 und 3, wobei der Ligand (1) und der Ligand (2) ein Hormon aus einer endokrinischen Drüse, ein Plasmaprotein, ein Virus-Antigen, eine bakterielle Species, ein α-Fetoprotein, ein carcinoembryonales Antigen, ein Hapten, ein erster Antikörper in dem Doppelantikörper-Verfahren oder ein Konjugat eines Antigen und eines ersten Antikörpers in dem Doppelantikörper-Verfahren ist.

7. Verfahren gemäß einem der Ansprüche 1 und 3 bis 6, wobei der Ligand (1) und der Ligand (2) dieselben Substanzen sind.

8. Verfahren gemäß einem jeden der vorangehenden Ansprüche, wobei der Antikörper ein Fragment von Immunoglobulin ist.

9. Verfahren gemäß einem jeden der Ansprüche 1 bis 7, wobei der Antikörper ein monoklonaler Antikörper ist.

10. Verfahren gemäß einem jeden der vorangehenden Ansprüche, wobei das Enzym ausgewählt ist aus $\alpha$-Amylase, Cellulase, Collagenase, Mannase, Protease, Elastase und Lipase.

11. Verfahren gemäß Anspruch 10, wobei die makromolekulare Substanz unlösliche Stärke ist im Falle von $\alpha$-Amylase als Enzym, ein Cellulose-Material ist im Falle von Cellulose als Enzym, Kollagen ist im Falle von Kollagenase als Enzym, Mannan ist im Falle von Mannase als Enzym, ein unlösliches Protein ist im Falle von Protease als Enzym, Elastin ist im Falle von Elastase als Enzym und ein Lipid ist im Falle von Lipase als Enzym.

12. Verfahren zur Messung von Theophyllin (1) in einer Probe von menschlichem Serum mit einem Gehalt an Amylase (2), umfassend das Inberührungbringen der Theophyllin enthaltenden Serumprobe, eines Antitheophyllin-IgG der Maus als Antikörper (3) gegen das Theophyllin, einer kovalent verbundenen Kombination von $\alpha$-Amylase und Theophyllin (4) und eines antihumanen Amylase-IgG der Ziege als Amylase-Inhibitor (5), dessen inhibierende Wirkung gegen die Amylase (2) im menschlichen Serum stärker ist als die gegenüber der $\alpha$-Amylase, das Reagieren vom Theophyllin in der Probe und dem kovalent mit der Amylase verbundenen Theophyllin sowie dem Amylase-Inhibitor (5) mit der Amylase (2), Digerieren von Stärke durch die $\alpha$-Amylase und Ermittlung der verbleibenden Aktivität der $\alpha$-Amylase und Verwendung der so erhaltenen Messung als Maß für den Theophyllin-Gehalt in der Probe vom menschlichen Serum.

FIG.1.

FIG.2.

FIG.3.

FIG.4.

FIG.5.